(19) Europäisches Patentamt
European Patent Office
Office européen des brevets

(11) Veröffentlichungsnummer: **0 257 629**
**A2**

(12) # EUROPÄISCHE PATENTANMELDUNG

(21) Anmeldenummer: **87112376.6**

(51) Int. Cl.⁴: **C07H 15/244**, A61K 31/70

(22) Anmeldetag: **26.08.87**

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 3093.

Patentansprüche für folgenden Vertragsstaaten: AT + GR + ES.

(30) Priorität: **28.08.86 DE 3629273**
**14.08.87 DE 3727139**

(43) Veröffentlichungstag der Anmeldung:
**02.03.88 Patentblatt 88/09**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Zeeck, Axel, Prof. Dr.**
**Brüder-Grimm-Allee 22**
**D-3400 Göttingen(DE)**
Erfinder: **Ciesiolka, Thomas**
**Gartemühle**
**D-3400 Göttingen(DE)**
Erfinder: **Zähner, Hans, Prof. Dr.**
**Im Hopfengarten 13**
**D-7400 Tübingen(DE)**
Erfinder: **Drautz, Hannelore, Dr.**
**Tulpenweg 16**
**D-7406 Mössingen(DE)**

(54) **Acylderivate von Urdamycin A, ihre Herstellung und ihre Verwendung.**

(57) Die O-Acylderivate von Urdamycin A zeigen antibiotische bzw. Antitumor-Aktivität.

EP 0 257 629 A2

# Acylderivate von Urdamycin A, ihre Herstellung und ihre Verwendung

In der Deutschen Offenlegungsschrift 3 441 933 werden die Urdamycine A bis F und ihre Aglykone, die entsprechenden Urdamycinone, beschrieben. Die Urdamycine werden durch Fermentation mit einem Mikroorganismenstamm, der aus einer Bodenprobe aus Tansania isoliert wurde, hergestellt. Der Mikroorganismus wurde als Streptomyces fradiae identifiziert und bei der Deutschen Sammlung von Mikroorganismen (DSM) unter der Nummer DSM 3093 hinterlegt. Die Urdamycinone kann man durch saure Hydrolyse oder Methanolyse der Urdamycine erhalten. Die bekannten Urdamycine und Urdamycinone wirken antibakteriell und tumorhemmend und können daher in Form pharmazeutischer Präparate zur Behandlung von bakteriell verursachten Infektionen oder zur Behandlung bei Tumorerkrankungen an Mensch und Tier verwendet werden.

Es wurde nun gefunden, daß die Acylderivate von Urdamycin A ebenfalls antibiotisch wirken und Antitumoraktivität aufweisen. Im Vergleich zu den bekannten Urdamycinen und Urdamycinonen zeigen diese neuen Derivate überraschend eine stärkere Aktivität.

Die Erfindung betrifft somit:

1. Die Verbindung der allgemeinen Formel I

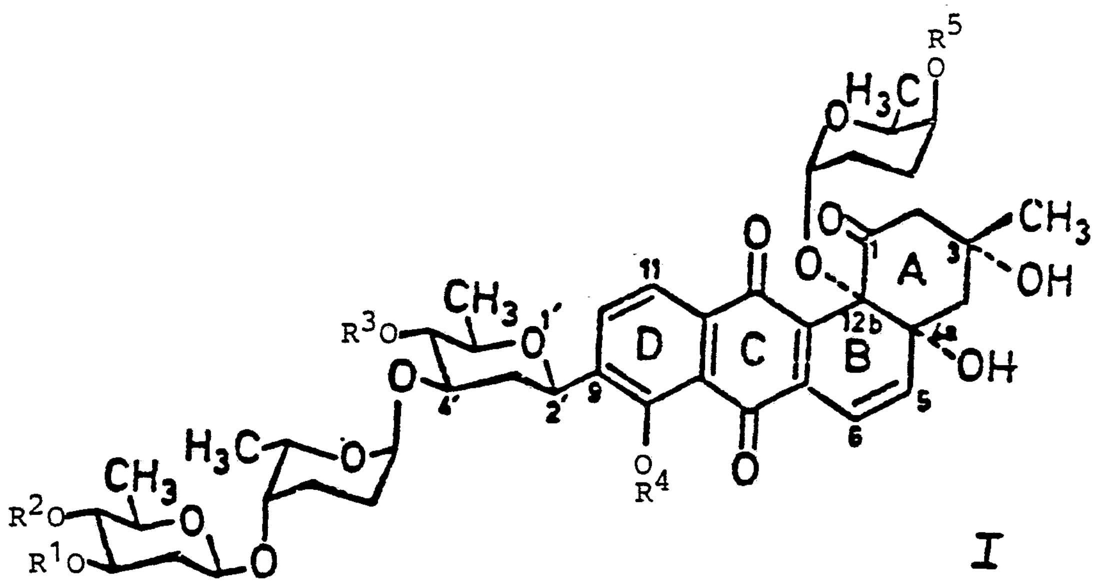

I

mit mindestens einer Acylgruppe,
in der $R^1$ bis $R^5$ unabhängig voneinander Wasserstoff oder eine ($C_1$-$C_{18}$)-Acylgruppe ist, wobei das 5′,3B,4B,4C,8-Penta-O-acetyl-urdamycin A ausgenommen ist.

2. Das Verfahren zur Herstellung der Verbindung der allgemeinen Formel I mit mindestens einer Acylgruppe, wobei das Penta-O-acetyl-urdamycin A ausgenommen ist, das dadurch gekennzeichnet ist, daß Urdamcyin A acyliert wird.

3. Die Verwendung der Verbindung der allgemeinen Formel I zur Herstellung von Heilmitteln.

Im folgenden wird die Erfindung, insbesondere in ihren bevorzugten Ausführungsformen detailliert beschrieben bzw. in den Patentansprüchen definiert.

In der Verbindung der allgemeinen Formel I kann $R^1$ bis $R^5$ Wasserstoff oder eine ($C_1$-$C_{18}$)-Acylgruppe bedeuten, die sich von organischen Carbonsäuren ableiten. Diese enthalten geradkettige, verzweigte oder cyclische aliphatische, aliphatisch-aromatische oder aromatische Kohlenwasserstoffreste, die ihrerseits unsubstituiert oder durch Halogen, z.B. Chlor oder Brom, veresterte oder veretherte Hydroxygruppen substituiert sind. Bevorzugt wird die ($C_1$-$C_{10}$)-Acylverbindung hergestellt, insbesondere die Acetylverbindung. Urdamycin A, das gemäß der Deutschen Offenlegungsschrift 3 441 933 erhältlich ist, wird dann mit einem Acylierungsmittel, das die entsprechenden Acylreste einer organischen Carbonsäure einführt, behandelt. Dabei verwendet man die entsprechenden Carbonsäure oder ein reaktionsfähiges Derivat, insbesondere ein Anhydrid. Die Acylierung kann in Gegenwart geeigneter Kondensationsmittel, bei Verwendung von freien Carbonsäuren,z.B. in Gegenwart von Carbodiimidverbindungen, wie Dicyclohexylcarbodiimid, oder Carbonylverbindungen, wie Diimidazolylcarbonyl, und bei Verwendung von reaktionsfähigen Carbonsäurederivaten, z.B. in Gegenwart von basischen Mitteln, wie Triniederalkylamin, z.B. Triethylamin, oder heterocyclischen Basen, z.B. Pyridin oder 4-Dimethylaminopyridin, oder basischen Salzen, z.B. wasserfreies Natriumacetat, durchgeführt werden.

Die Acylierungsreaktion kann in Abwesenheit oder in Gegenwart eines Lösungsmittels oder Lösungsmittelgemisches unter Kühlen, bei Raumtemperatur oder unter Erwärmen und, wenn notwendig, in einem geschlossenen Gefäß und/oder in einer Inertgas-, z.B. Stickstoffatmosphäre, durchgeführt werden. Geeignete Lösungsmittel sind beispielsweise einfache oder substituierte, z.B. chlorierte, aliphatische, cycloaliphatische oder aromatische Kohlenwasserstoffe, wobei man geeignete Veresterungsreagentien, wie Acetanhydrid, aber auch geeignete Basen, wie Pyridin, als Verdünnungsmittel verwenden kann. Durch Wahl entsprechender Reaktionsbedingungen kann die Synthese bestimmter Acylverbindungen gezielt gesteuert werden. Dies erreicht man beispielsweise durch Variation der Base, der Reaktionstemperatur oder der Reaktionszeit bzw. durch Variation von mehreren Parametern gleichzeitig.

Bevorzugt wird die Reaktion jedoch mit einem Anhydrid, insbesondere Acetanhydrid, in Gegenwart von Pyridin oder Natriumacetat durchgeführt. Die Reaktionstemperaturen und die Reaktionszeiten liegen zwischen -10 und +100°C und 2 Minuten bis 48 Stunden, bevorzugt zwischen 20 bis 30°C und 8 Minuten bis 16 Stunden. Das Verhältnis Anhydrid zu Basen liegt im Bereich von 20:1 bis 1:1, bevorzugt bei 2:1. Die Konzentration von Urdamycin A im Reaktionsansatz liegt zwischen 0.05-10 %, bevorzugt bei 0.1-1 %.

Nach Beendigung der Reaktion kann das Reaktionsprodukt durch Extraktion isoliert werden. Zur weiteren Reinigung wird es dann beispielsweise an Kieselgel chromatographiert.

Alternativ können Mono-bis Tetraacylverbindungen durch basische Acylspaltung von 5',3B,4B,4C,8-Penta-O-acyl-urdamycin A hergestellt werden. Verwendung finden Basen, wie die Alkali-oder Erdalkalihydroxide oder Alkalicarbonate, in wäßrig/alkoholischer Lösung bei -10 bis +100°C. Die Acylspaltung wird bevorzugt mit einer gesättigten Natriumcarbonat-Lösung in wäßrig/methanolischer Lösung bei 25°C durchgeführt. Die anschließende Aufarbeitung erfolgt wie oben erwähnt.

In den folgenden Beispielen wird die Erfindung in weiteren Einzelheiten beschrieben. Prozentangaben beziehen sich, wenn nicht anders angegeben, auf das Gewicht.

## Beispiele

1. Herstellung von 5', 3B, 4B, 8-Tetra-O-acetyl-Urdamycin A

129 mg Urdamycin A, das gemäß der Deutschen Offenlegungsschrift 3 441 933, Beispiel 4, erhalten wurde, wurden in 22,5 ml einer Mischung aus Acetanhydrid/Pyridin im Verhältnis 2:1 gelöst und 7 Stunden bei Raumtemperatur gerührt. Es wurde anschließend auf Eis gegossen, 3 mal mit Chloroform extrahiert und der Extrakt zur Trockne eingedampft. Pyridinreste entfernte man durch mehrmalige Aufnahme in Toluol mit anschließendem Eindampfen. Es wurde an Kieselgel (Kieselgel 60 unter 0.08 mm, Macherey-Nagel) (Säule 2x30 cm, Chloroform/Methanol 100:2, V:V) chromatographiert. Man erhält neben 148 mg (92%) 5',3B,4B,4C,8-Penta-O-acetyl-urdamycin A 11 mg (6 %) der gewünschten Acetylverbindung als gelben Feststoff, der wie folgt charakterisiert wurde:

$C_{51}H_{64}O_{21}$ (1013,1)
Schmp.: 160°C (Zersetzung)
IR (KBr): 3450; 2980; 2938; 2870; 1781; 1745; 1664; 1632; 1598; 1561 $cm^{-1}$
UV Methanol : $\lambda_{max}$ ($\epsilon$) = 360 (4770); 313 sh (5510); 257,7 (15280) nm
UV (Methanol/HCl) : $\lambda_{max}$ ($\epsilon$) = wie Methanol
UV (Methanol/NaOH) : $\lambda_{max}$ ($\epsilon$) = 401 (3140); 315,7 (7660); 255 (12560); 224 (16980) nm
$^1H$-NMR (200 MHz, $CDCl_3$): siehe Tabelle 1
CD (Methanol): $\lambda_{extr.}$ ($\Theta^{20} x 10^{-3}$) = 401 (-5,1); 318 (9,7); 288 (7,9); 267 (1,0); 232 (18,0) nm

2. Herstellung von 3B,4B,8-Tri-O-acetyl-urdamycin A

Man löste 25 mg Urdamcyin A in 7,5 ml eines Gemisches aus Acetanhydrid/Pyridin (2:1) und ließ 8 Minuten bei Raumtemperatur rühren, wobei sich die Lösung von orange nach gelb verfärbte. Da sich alles Urdamycin A umgesetzt hatte (DC), goß man auf Eis und extrahierte 2 mal mit je 30 ml Chloroform. Man dampfte zur Trockene ein und entfernte Pyridinreste durch mehrmaliges Aufnehmen und Eindampfen mit Toluol. Man chromatographierte an Kieselgel (Säule 2 x 50 cm, Chloroform/Methanol 98:2; V:V) und eluierte folgende Zonen (von unten gezählt):

1. Mischfraktion aus mindestens zwei Substanzen, 9.4 mg, gelb;
2. DC-einheitliches Hauptprodukt, 15 mg, gelb.

Man reinigte die 2. Zone an ®Sephadex LH 20 (Säule 2.5 x 100 cm, Methanol) nach und erhielt 14 mg eines gelben Feststoffes.

Das $^1$H-NMR-Spektrum zeigte, daß es sich bei dem Feststoff um ein Gemisch aus
80 % 3B,4B,8-Tri-O-acetyl-urdamycin A und
20 % 3B,4B-Di-O-acetyl-urdamycin A
handelt. Die $^1$H-NMR-spektroskopischen Daten sind in der Tabelle 1 wiedergegeben.

3. Herstellung von 3B-O-Acetyl-urdamycin A

Man löste 36 mg Urdamycin A in 10 ml Acetanhydrid und fügte eine Spatelspitze wasserfreies Natriumacetat hinzu. Nach 2 Stunden brach man die Reaktion ab, indem man auf Eis goß. Man ließ 4 Stunden rühren und extrahierte 4 mal mit je 50 ml Chloroform, dampfte am Rotationsverdampfer zur Trockene ein und chromatographierte den festen Rückstand an Kieselgel (Säule 2 x 50 cm, Methylenchlorid/Ethanol 9:1; V:V). Man erhielt folgende Zonen (von unten gezählt):

1. Mischfraktion aus zwei Substanzen, 2 mg, orange;
2. DC-einheitliches 3B-O-Acetyl-urdamycin A, 21 mg, orange;
3. Mischfraktion aus zwei Substanzen, 3 mg, orange;
4. nicht umgesetztes Urdamcyin A, 12 mg, orange;

Das so erhaltene 3B-O-Acetyl-urdamycin wurde, ebenso wie das nicht umgesetzte Urdamycin A, an Sephadex LH 20 (Säule 2,5 x 100 cm Methanol) nachgereinigt. Man erhielt neben 11 mg Urdamycin A noch 20 mg 3B-O-Acetyl-urdamycin A (53 %, 76 % bezogen auf den Umsatz) als orangen Feststoff der wie folgt charakterisiert wurde:

$C_{45}H_{58}O_{18}$ (886,9)
Ber. C 60,82 H 6,53
Gef. C 60,83 H 6,70
Schmp. 178°C (Zersetzung)
IR (KBr): 3430; 2980; 2935; 1727; 1660 sh; 1640; 1621; 1565 $cm^{-1}$
UV (Methanol : $\lambda_{max}$ ($\epsilon$) = 425,7 (3440); 320,2 (2670); 240 sh (11710) nm
UV (Methanol/HCl) : $\lambda_{max}$ ($\epsilon$) = wie Methanol
UV (Methnaol/NaOH) : $\lambda_{max}$ ($\epsilon$) = 585,5 (3340); 403,7 (1520); 324,5 (5470); 228,7 (18150) nm
$^1$H-NMR (200 MHz, $CDCl_3$): siehe Tabelle 2
CD (Methanol: $\lambda_{extr.}$ ($\Theta^{20}$x$10^{-3}$) = 492 (-3,0); 445 (2,0);

4. Herstellung von 3B,4B-Di-O-acetyl-urdamycin A

Man löste 50 mg Urdamycin A in 15 ml Acetanhydrid und versetzte mit einer Spatelspitze wasserfreiem Natriumacetat. Nach 16 Stunden hatte sich alles Urdamycin A umgesetzt (DC). Man goß auf Eis und ließ 3 Stunden hydrolysieren, extrahierte 3 mal mit je 50 ml Chloroform und dampfte zur Trockene ein. Man chromatographierte an Kieselgel (Säule 2 x 30 cm, Methylenchlorid/Ethanol 95:5; V:V) und erhielt folgende Zonen (von unten gezählt):

1. Mischfraktion aus mindestens vier Substanzen, 15 mg, gelb;
2. DC-einheitliches 3B-O-Acetyl-urdamycin A, 12 mg, orange;
3. Mischfraktion aus mindestens drei Substanzen, 9 mg, orange;
4. DC-einheitliches 3B,4B-Di-O-acetyl-urdamycin A, 12 mg, orange.

Die Zonen 2. und 4. wurden an Sephadex LH 20 (Säule 2.5 x 100 cm, Methanol) nachgereinigt. Man erhielt

10 mg 3B-O-Acetyl-urdamycin A (19%) als orangen Feststoff und

10 mg 3B,4B-Di-O-acetyl-urdamycin A (18%) als orangen Feststoff. Der letztere wurde wie folgt charakterisiert:

$C_{47}H_{60}O_{19}$ (929,0)

Ber. C 60,71 H 6,46

Gef. C 60,63 H 6,69

Schmp.: 169°C

IR (KBr): 3440; 2980; 2935; 2870; 1745; 1735; 1658; 1640; 1621; 1563 $cm^{-1}$

UV (Methanol) :$\lambda_{max}$ ($\epsilon$) = 426 (4070); 320,5 (3050); 240 sh (14870) nm

UV (Methanol/HCl) :$\lambda_{max}$ ($\epsilon$) = wie Methanol

UV (Methanol/NaOH);$\lambda_{max}$ ($\epsilon$) = 581,5 (3860); 404,5 (1470); 324,7 (6500); 228,5 (22820) nm

$^1$H-NMR (200 MHz, $CDCl_3$): siehe Tabelle 2

CD (Methanol): $\lambda_{extr.}$ ($\Theta^{20}$x$10^{-3}$) = 466 (3,6); 406 (-8,6); 331 (29,0); 292 (10,7); 263 (-3,6); 236 sh (30,5); 226 (34,0) nm

5. Herstellung von 5',3B,4B,4C-Tetra-O-acetyl-urdamycin A und 4B,4C-Di-O-acetyl-urdamycin A

Man löste 73 mg 5',3B,4B,4C,8-Penta-O-acetyl-urdamycin, das gemäß der Europäischen Patentanmeldung 3 441 933, Beispiel 14, hergestellt wurde, in 7 ml Wasser und 10 ml Methanol und versetzte bei 25°C mit 3,5 ml einer gesättigten Natriumcarbonatlösung. Die Lösung färbte sich sofort tiefblau. Man ließ unter Stickstoffatmosphäre 10 min. rühren und brach die Reaktion durch Zugabe von Acetanhydrid ab (Farbumschlag). Man goß die Lösung auf Eis und extrahierte fünf mal mit je 30 ml Chloroform. Anschließend engte man am Rotationsverdampfer bis zur Trockene ein und chromatographierte den festen Rückstand an Kieselgel (Säule 2,5 x 50 cm, Methylenchlorid/Ethanol 9:1; V:V). Man eluierte folgende Zonen (von unten gezählt):

1. nicht umgesetztes Penta-O-acetyl-urdamycin A, 10 mg, gelb;
2. Mischfraktion aus 5',3B,4B,4C-Tetra-O-acetyl-urdamycin A und Penta-O-acetyl-urdamycin A, 9 mg, orange;
3. DC-einheitliches 5',3B,4B,4C-Tetra-O-acetyl-urdamycin A 38 mg, orange;
4. DC-einheitliches 4B,4C-Di-O-acetyl-urdamycin A, 11 mg, orange.

Die 3. und die 4. Zonen wurden an Sephadex LH 20 (Säule 2,5 x 100 cm, Methanol) nachgereinigt. Man erhielt:

-31 mg (35 %, 41% bezogen auf den Umsatz) 5',3B,4B,4C-Tetra-O-acetyl-urdamycin A als orangen Feststoff

-10 mg (12 %, 14 % bezogen auf den Umsatz) 4B,4C-Di-O-acetyl-urdamycin A als orangen Feststoff.

a) 5',3B,4B,4C-Tetra-O-acetyl-urdamycin A

$C_{51}H_{64}O_{21}$ (1013,1)

Ber. C 60,41 H 6,32

Gef. C 60,42 H 6,50

Schmp. 184°C

IR (KBr): 3440; 2940; 1740; 1660 sh; 1640; 1625; 1565 $cm^{-1}$

UV (Methanol) : $\lambda_{max}$ ($\epsilon$) = 427,2 (4270); 326,5 (3560); 240 sh (15970) nm

UV (Methanol/HCl : $\lambda_{max}$ ($\epsilon$) = wie Methanol

UV (Methanol/NaOH) : $\lambda_{max}$ ($\epsilon$) = 577 (4020); 401,5 (1860); 323,5 (7330), 229,7 (23790)nm

$^1$H-NMR (200 MHz, $CDCl_3$): Tabelle 2

CD (Methanol); $\lambda_{extr.}$ ($\Theta^{20}$x$10^{-3}$) = 500 (-4,0); 455 (3,5); 405 (-9,0); 325 (29,6); 296 sh(20,1); 263 (-5,0); 232 (35,1) nm

b) 4B,4C-Di-O-acetyl-urdamycin A

$C_{47}H_{60}O_{19}$ (929,0)
Ber. C 60,71 H 6,46
Gef. C 60,80 H 6,45
Schmp. 175-181 °C
IR (KBr): 3450; 1740; 1660; 1640; 1622; 1565 $cm^{-1}$
UV (Methanol) : $\lambda_{max}$ ($\epsilon$) = 427 (4100); 326 (3900) nm
UV (Methanol/HCl) : $\lambda_{max}$ ($\epsilon$) = wie Methanol
UV (Methanol/NaOH): $\lambda_{max}$ ($\epsilon$) = 585 (33700); 406 (1600); 324 (8300); 228 (21700) nm
$^1$H-NMR (200 MHz, $CDCl_3$): Tabelle 2
CD (Methanol: $\lambda_{extr.}$ ($\Theta^{20}x10^{-3}$) = 500 (-4,8); 455 (5,7); 408 (-3,8); 325 (23,5); 295sh (16,8); 263 (-4,3); 235 (31,1) nm

Tabelle 1: $^1$H-NMR-Signale der Acetate (1) und (2) in $CDCl_3$
($\delta$ in ppm rel. zu internem TMS, J in Hz)

| | (1) | (2) |
|---|---|---|
| $2-H_a$ | 2.53 (d, 13) | 2,52 (d, 13) |
| $2-H_e$ | 2.81 (d, 13;2) | 2.79 (dd, 13;2) |
| $3-CH_3$ | 1.25 (s) | 1.23 (s) |
| $4-H_2$ | 1.2-2.2 (komplex) | 1.3-2.0 (komplex) |
| 5-H | 6.01 (d, 10) | 6.38 (d, 10) |
| 6-H | 6.66 (d, 10) | 6.84 (d, 10) |
| 10-H | 8.13 (d, 8) | 8.10 (d, 8) |
| 11-H | 8.02 (d, 8) | 8.01 (d, 8) |
| 2'-H | 4.95 (verd.) | 4.89 (dd, 10;2) |
| $3'-H_2$ | 1.2-2.2 (komplex) | 1.3-2.0 (komplex) |
| 4'-H | 3.98 (m) | 3.4-3.8 (komplex) |
| 5'-H | 4.73 (dd, 9;9) | 3.17 (dd, 9;9) |
| 6'-H | 3.61 (dq, 9;6) | 3.4-3.8 (komplex) |
| $6'-CH_3$ | 1.27 (d, 6) | 1.41 (d, 6) |
| 1A-H | 4.91 (s) | 5.01 (s, breit) |
| $2A-H_2$ | 1,2-2.2 (komplex) | 1.3-2.0 (komplex) |
| $3A-H_2$ | 1.2-2.2 (komplex) | 1.3-2.0 (komplex) |
| 4A-H | 3.48 (s, breit) | 3.4-3.8 (komplex) |
| 5A-H | 3.88 (dq, 6.5;2) | 4.12 (dq, 6.5;2) |
| $5A-CH_3$ | 1.13 (d, 6.5) | 1.18 (d, 6) |
| 1B-H | 4.57 (dd, 9.5;2) | 4.57 (dd, 10;2) |
| $2B-H_a$ | 1.2-2.2 (komplex) | 2.18 (ddd, 13;12;12) |
| $2B-H_a$ | 2.39 (ddd, 13;5;2) | 2.38 (ddd, 13;5;2) |
| 3B-H | 4.95 (m) | 4.75 (m) |
| 4B-H | 4.81 (dd, 9;9) | 4.48 (dd, 9;9) |
| 5B-H | 3.44 (dq, 9;6) | 3.4-3.8 (komplex) |
| $5B-CH_3$ | 1.14 (d, 6) | 1.21 (d, 6) |
| 1C-H | 5.44 (s, breit) | 5.41 (s, breit) |
| $2C-H_2$ | 1.2-2.2 (komplex) | 1.3-2.0 (komplex) |
| $3C-H_2$ | 1.2-2.2 (komplex) | 1.3-2.0 (komplex) |
| 4C-H | 3.49 (s, breit) | 3.4-3.8 (komplex) |
| 5C-H | 3.69 (dq, 6.5;2) | 3.67 (dq, 6.5;2) |
| $5C-CH_3$ | 0.57 (d, 6.5) | 0.57 (d, 6.5) |

5',3B,4B,8-Tetra-O-acetyl-urdamycin A (1)
3B,4B,8-Tri-O-acetyl-urdamycin A (2)

Tabelle 6: $^{1}$H-NMR-Signale der Acetate (3) bis (6) in $CDCl_3$
(δ in ppm rel. zu internem TMS, J in Hz)

| | (3) | (4) | (5) | (6) |
|---|---|---|---|---|
| 2-$H_a$ | 2.51 (d, 13) | 2.52 (d, 13) | 2.42 (d, 13) | 2.51 (d, 13) |
| 2-$H_e$ | 2.79 (dd, 13;2.5) | 2.79 (dd, 13;2) | 2.82 (dd, 13;2) | 2.82 (dd, 13;2) |
| 3-$CH_3$ | 1.24 (s) | 1.23 (s) | 1.24 (s) | 1.25 (s) |
| 4-$H_2$ | 1.4-2.0 (komplex) | 1.3-2.0 (komplex) | 1.2-2.2 (komplex) | 1.2-2.0 (komplex) |
| 5-H | 6.43 (d, 10) | 6.24 (d, 10) | 6.45 (d, 10) | 6.46 (d, 10) |
| 6-H | 6.91 (d, 10) | 6.92 (d, 10) | 6.93 (d, 10) | 6.95 (d, 10) |
| 10-H | 7.95 (d, 8) | 7.94 (d, 8) | 7.95 (d, 8) | 7.96 (d, 8) |
| 11-H | 7.70 (d, 8) | 7.70 (d, 8) | 7.72 (d, 8) | 7.72 (d, 8) |
| 2'-H | 4.88 (dd, 11.5;2) | 4.88 (dd, 10;2) | 4.92 (dd, 11;2) | 4.91 (dd, 10;2) |
| 3'-$H_a$ | 1.4-2.0 (komplex) | 1.3-2.0 (komplex) | 1.2-2.2 (komplex) | 1.2-2.0 (komplex) |
| 3'-$H_e$ | 2.18 (ddd, 13;5;2) | 2.18 (ddd, 13;5;2) | 2.32 (ddd, 13;5;2) | 2.36 (ddd, 13;5;2) |
| 4'-H | 3.70 (m) | 3.4-3.6 (komplex) | 4.01 (m) | 4.00 (m) |
| 5'-H | 3,15 (dd, 9;9) | 3.17 (dd, 9;9) | 3.10 (ddd, 9;9;6) | 4.50 (dd, 9;9) |
| 6'-H | 3.56 (m) | 3.4-3.6 (komplex) | 3.61 (dq, 9;6) | 3.6-3.8 (komplex) |
| 6'-$CH_3$ | 1.43 (d, 6) | 1.41 (d, 6) | 1.30 (d, 6) | 1.30 (d. 6) |
| 1A-H | 5.02 (d, 2) | 5.03 (d, 2) | 4.99 (d, 2) | 4.93 (d, 2) |
| 2A-$H_2$ | 1.4-2.0 (komplex) | 1.3-2.0 (komplex) | 1.2-2.2 (komplex) | 1.2-2.0 (komplex) |
| 3A-$H_2$ | 1.4-2.0 (komplex) | 1.3-2.0 (komplex) | 1.2-2.2 (komplex) | 1.2-2.0 (komplex) |
| 4A-H | 3.44 (s) | 3.4-3.8 (komplex) | 3.49 (s) | 3.60 (s) |
| 5A-H | 4.12 (dq, 6.5;2) | 4.12 (dq, 6.5;2) | 3.89 (6.5;2) | 3.91 (dq, 6.5;2) |
| 5A-$CH_3$ | 1.21 (d, 6.5) | 1.18 (d, 6) | 1.20 (d, 6) | 1.18 (d, 6) |
| 1B-H | 4.55 (dd, 10;2) | 4.57 (dd, 10;2) | 4.52 (dd, 10;2) | 4.57 (dd, 9;2) |
| 2B-$H_a$ | 1.4-2.0 (komplex) | 1.3-2.0 (komplex) | 1.2-2.2 (komplex) | 2.16 (verd.) |
| 2B-$H_e$ | 2.39 (ddd, 13;5;2) | 2.38 (ddd, 13;5;2) | 2.62 (sss, 13;5;2) | 2.63 (ddd, 13;5;2) |
| 3B-H | 4.79 (ddd, 12;8;5) | 4.97 (m) | 3.5-3.8 (komplex) | 4.8 (m) |
| 4B-H | 3.44 (dd, 9;9) | 4.74 (dd, 9;9) | 4.83 (dd, 9;9) | 4.79 (dd, 9.5;9.5) |
| 5B-H | 3.29 (m) | 3.4-3.8 (komplex) | 3.27 (dq, 9;6) | 3.30 (dq, 9;6) |
| 5B-$CH_3$ | 1.33 (d, 6) | 1.21 (d, 6) | 1.28 (d, 6) | 1.20 (d, 6) |
| 1C-H | 5.41 (s) | 5.40 (s) | 5.47 (s) | 5.60 (s) |
| 2C-$H_2$ | 1.4-2.0 (komplex) | 1.3-2.0 (komplex) | 1.2-2.2 (komplex) | 1.2-2.2 (komplex) |
| 3C-$H_2$ | 1.4-2.0 (komplex) | 1.3-2.0 (komplex) | 1.2-2.2 (komplex) | 1.2-2.0 (komplex) |

| | | | | |
|---|---|---|---|---|
| 4C-H | 3.31 (s) | 3.4-3.8 (komplex) | 4.70 (o, 3x2) | 4.67 (s) |
| 5C-H | 3.67 (dq, 6.5;2) | 3.67 (dq, 6.5;2) | 3.69 (dq, 6.5;2) | 3.6-3.8 (komplex) |
| 5C-$CH_3$ | 0.58 (d, 6.5) | 0.57 (d, 6.5) | 0,52 (d, 6.5) | 0.53 (d, 6.5) |

3B-O-Acetyl-urdamycin A (3)
3B,4B-Di-O-acetyl-urdamycin A (4)
4B,4C-Di-O-acetyl-urdamycin A (5)
5',3B,4B,4C-Tetra-O-acetyl-urdamycin A (6)

6. In vitro Test auf cytostatische Aktivität

Der Versuch wurde nach dem Verfahren von Hamburger und Salmon [(New Engl.J.Med. 298, 1321-1327 (1978)] durchgeführt. Das Medium wurde durch McCoy 5A ersetzt und die Zellzahl auf $5x10^2$ Zellen/Platte reduziert.

a) Wirkung von L 1210 Leukämie-Zellen im kontinuierlichen Experiment

Es wurde eine kontinuierliche Inkubation der Zellen mit verschiedenen Konzentrationen der Testsubstanz aus den Beispielen 1 bis 5 sowie 5',3B,4B,4C,8-Penta-O-acetyl-urdamycin A vorgenommen. Die zu testenden Verbindungen wurden vor dem Ausplattieren der Zellkulturen auf die Agar-Platten aufgetragen. Die Zellkulturen wuchsen dann 5 bis 7 Tage im Inkubator in einer Atmosphäre von 5 % $CO_2$, 20 % $O_2$ und 95 % relativer Luftfeuchte bei 37°C. Nach dieser Zeit wurde die Anzahl an Zellkolonien mit einem Durchmesser ab 60 µm relativ zu der Anzahl an Zellkolonien, die im Vergleich ohne Testsubstanz gewachsen sind, bestimmt (in %).

b) Wirkung auf L 1210 Leukämie Zellen im 1 Stunden-Experiment:

Im Test wurden die Zellen mit unterschiedlicher Konzentration der Testsubstanz 1h bei 37°C inkubiert. Danach wurden die Zellen zweimal mit McCoy 5A-Lösung gewaschen und anschließend auf Agar-Platten gemäß der Methode von Hamburger und Salmon aufgetragen. Anschließend kultivierte man die Zellen wie oben beschrieben und bestimmte die Anzahl der Zellkolonien (Auswertung wie in a).

Nach der Dosis/Wirkungskurve wurde der $IC_{50}$-Wert für die kontinuierliche und die 1-stündige Inkubation bestimmt.

c) Proliferations-Versuch

Tumor Zellen L 1210 der exponentialen Wachstumsphase [$5x10^3$ Zellen/ml Roswell-Park-Memorial-Institute-Medium (RPMI)] wurden in einer Mikrotiter Platte mit 26 Vertiefungen über einen Zeitraum von 72 Stunden bei 37°C, 5 % $CO_2$ und 95 % relativer Luftfeuchte mit verschiedenen Konzentrationen der Testsubstanzen aus den Beispielen 1 bis 5 sowie 5',3B,4B,4C,8-Penta-O-acetyl-urdamycin A inkubiert. Nach 65 Stunden wurden 50 µl 3-(4,5-Dimethylthiazol-2-yl)-2,5-diphenyl-tetrazoliumbromid (MTT) (2,5 mg/ml MTT in Phosphate Buffer Saline (PBS) hinzugegeben. Das MTT wurde von lebenden Zellen in den roten wasserunlöslichen Farbstoff Formazan reduziert. Nach weiteren 7 Stunden wurde das überstehende Medium vorsichtig entfernt. Formazan wurde durch Zugabe von 100 µl Dimethylsulfoxid pro Vertiefung und anschließendes leichtes Schütteln in Lösung bebracht. Die Extinktion jeder Vertiefung wurde mit einem Multisan-Photometer 340 CC, (Fa. Flow) bei 491 nm bestimmt. Die Ergebnisse wurden aus dem Extinktionsverhältnis von Zellen mit Testsubstanz zu Zellen ohne Testsubstanz ermittelt.

Zu allen Fällen wurden die Versuche in 4-facher Ausführung durchgeführt. Die Abweichung der Ergebnisse betrug weniger als 15 %.

Die Ergebnisse sind in der folgenden Tabelle zusammengefaßt:

| Testsubstanz | Proliferations-Test | $IC_{50}$ (µg/ml) Stamm -Test kontinuierlich | 1 h |
|---|---|---|---|
| Penta-O-acetyl-U. A | < 1 | 0,042 | 0,12 |
| 5',3B,4B,8-Tetra-O-acetyl-U. A | < 1 | 0,075 | - |
| 5',3B,4B,4C-Tetra-O-acetyl-U. A | < 1 | 0,078 | 0,25 |
| 4B,4C-Di-O-acetyl-U. A | < 1 | 0,13 | 0,85 |
| 3B,4B-Di-O-acetyl-U. A | < 1 | 0,11 | 0,35 |
| 3B,4B,8-Tri-O-acetyl-U. A | < 1 | 0,4 | 1,1 |
| 3B-O-Acetyl-urdamycin A | < 1 | 0,28 | 2,2 |
| 3B-O-Octanoyl-urdamycin A | < 1 | n.b. | 0,26 |
| 4B-O-Octanoyl-urdamycin A | < 1 | n.b. | 0,09 |

n.b. = nicht bestimmt

## Ansprüche

1. Die Verbindung der allgemeinen Formel I

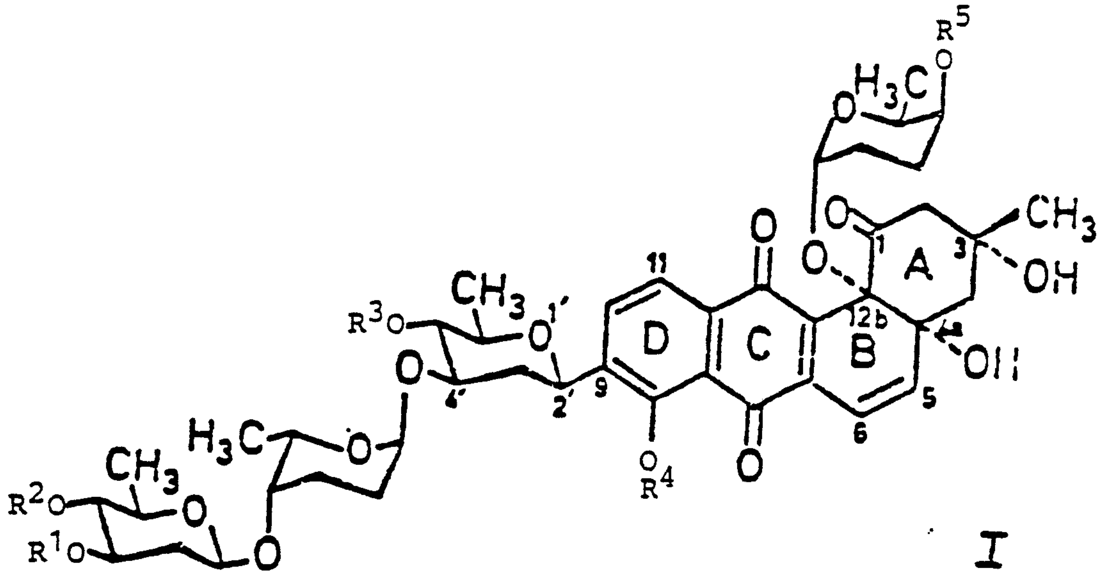

I

in der $R^1$ bis $R^5$ unabhängig voneinander Wasserstoff oder eine ($C_1$ bis $C_{18}$)Acylgruppe ist, wobei mindestens eine Acylgruppe vorliegt und das 5',3B,4B,4C,8-Penta-O-acetyl-urdamycin A ausgenommen ist.

2. Die Verbindung nach Anspruch 1, dadurch gekennzeichnet, daß $R^1$ bis $R^5$ unabhängig voneinander Wasserstoff oder eine ($C_1$ bis $C_{10}$)-Acylgruppe bedeutet.

3. Die Verbindung nach Anspruch 2, dadurch gekennzeichnet, daß $R^1$ bis $R^5$ unabhängig voneinander Wasserstoff oder eine Acetylgruppe bzw. Octanoylgruppe bedeutet.

4. Verfahren zur Herstellung der Verbindung der allgemeinen Formel I mit mindestens einer Acylgruppe, wobei das 5',3B,4B,4C,8-Penta-O-acetyl-urdamycin A ausgenommen ist,

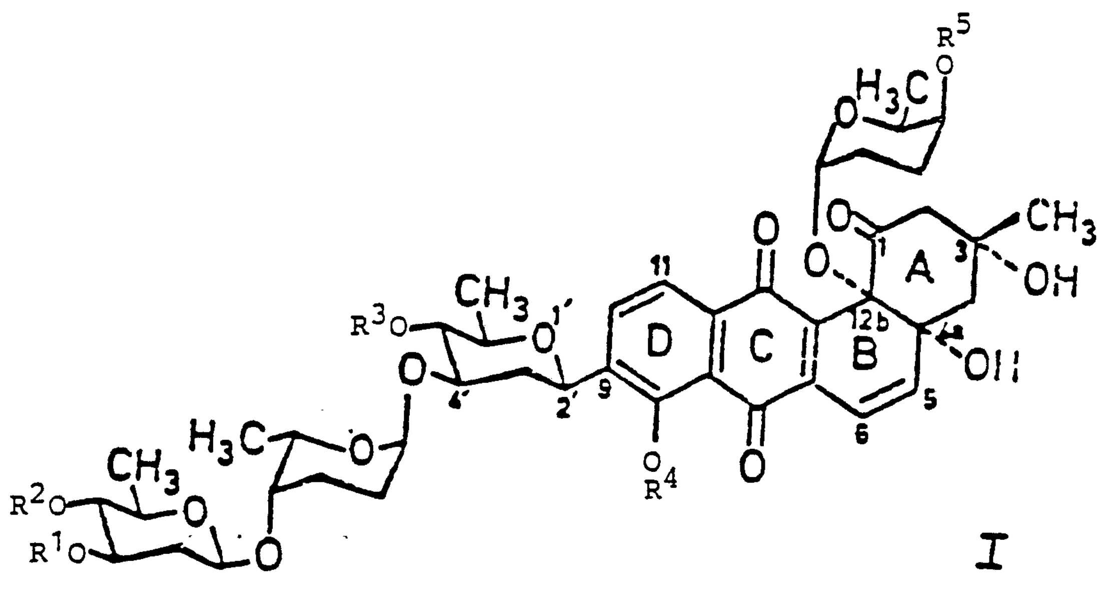

I

in der $R^1$ bis $R^5$ unabhängig voneinander Wasserstoff oder eine ($C_1$ bis $C_{18}$)-Acylgruppe ist, dadurch gekennzeichnet, daß Urdamycin A acyliert wird.

5. Verfahren nach Anspruch 4, dadurch gekennzeichnet, daß Urdamycin A mit ($C_1$-$C_{10}$)-Acylgruppen acyliert wird.

6. Verfahren nach Anspruch 4 und 5, dadurch gekennzeichnet, daß Urdamycin A mit dem Anhydrid der Acylverbindung in Gegenwart einer Base acyliert wird.

7. Verfahren nach Anspruch 6, dadurch gekennzeichnet, daß Urdamycin A mit Acetanhydrid in Gegenwart von Pyridin oder Natriumacetat acyliert wird.

8. Verfahren zur Herstellung der Verbindung der allgemeinen Formel I mit mindestens einer Acylgruppe, wobei das 5',3B,4B,4C,8-Penta-O-acetyl-urdamycin A ausgenommen ist,

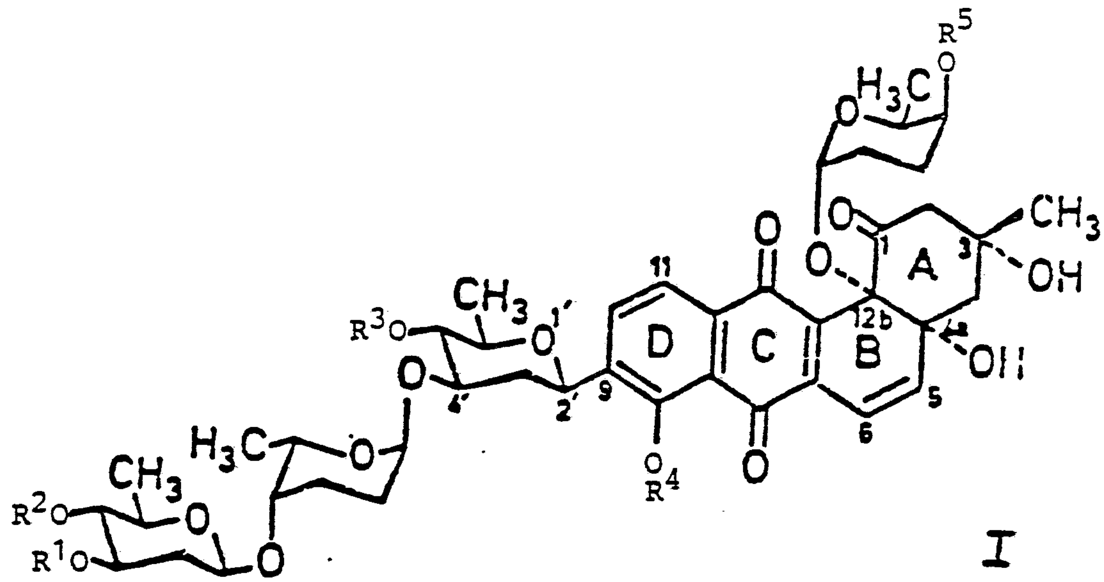

I

in der $R^1$ bis $R^5$ unabhängig voneinander Wasserstoff oder eine ($C_1$ bis $C_{18}$)-Acylgruppe ist, dadurch gekennzeichnet, daß 5',3B,4B,4C,8-Penta-O-acyl-urdamycin A basisch gespalten wird.

9. Verfahren nach Anspruch 8, dadurch gekennzeichnet, daß Penta-O-acyl-urdamycin A mit einer gesättigten Natriumcarbonatlösung in wäßrig/methanolischer Lösung gespalten wird.

10. Verwendung der Verbindung der allgemeinen Formel I nach einem oder mehreren der Ansprüche 1 bis 3 zur Herstellung von Heilmitteln.

11. Verwendung der Verbindung nach Anspruch 10, zur Herstellung von Heilmitteln zur Behandlung von infektiösen Krankheiten und Tumoren.

12. Verwendung der Verbindung der allgemeinen Formel I nach einem oder mehreren der Ansprüche 1 bis 3 als antibiotisches Agens.

Patentansprüche für den folgenden Vertragsstaaten: Griechenland, Spanien und Österreich:

1. Verfahren zur Herstellung der Verbindung der allgemeinen Formel I mit mindestens einer Acylgruppe, wobei das 5',3B,4B,4C,8-Penta-O-acetyl-urdamycin A ausgenommen ist,

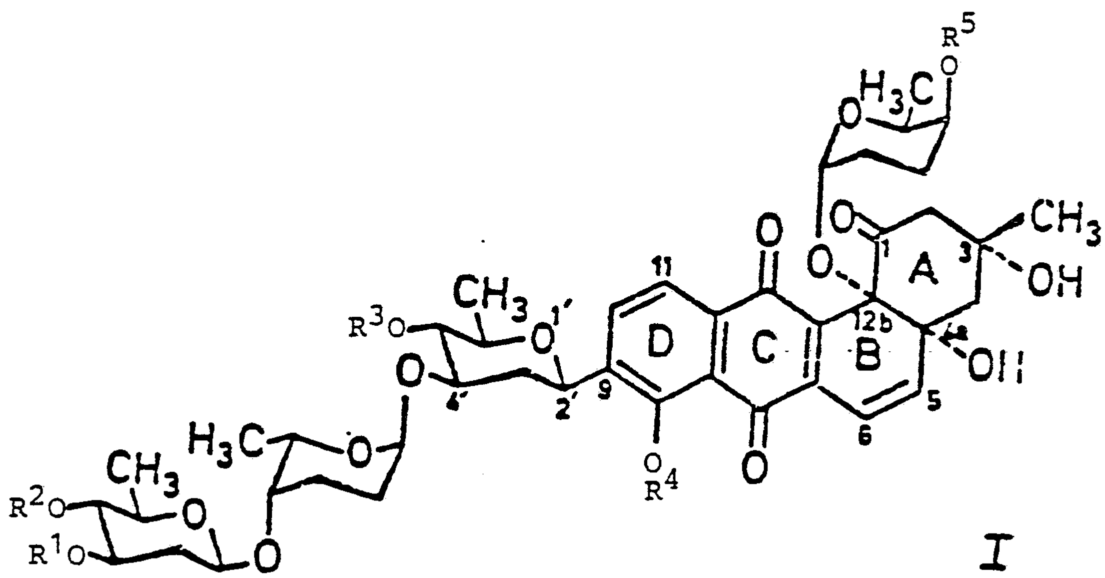

I

in der $R^1$ bis $R^5$ unabhängig voneinander Wasserstoff oder eine ($C_1$ bis $C_{18}$)-Acylgruppe ist, dadurch gekennzeichnet, daß Urdamycin A acyliert wird.

2. Verfahren nach Anspruch 1, dadurch gekennzeichnet, daß Urdamycin A mit ($C_1$-$C_{10}$)-Acylgruppen acyliert wird.

3. Verfahren nach Anspruch 1 und 2, dadurch gekennzeichnet, daß Urdamycin A mit dem Anhydrid der Acylverbindung in Gegenwart einer Base acyliert wird.

4. Verfahren nach Anspruch 3, dadurch gekennzeichnet, daß Urdamycin A mit Acetanhydrid in Gegenwart von Pyridin oder Natriumacetat acyliert wird.

5. Verfahren zur Herstellung der Verbindung der allgemeinen Formel I mit mindestens einer Acylgruppe, wobei das 5',3B,4B,4C,8-Penta-O-acetyl-urdamycin A ausgenommen ist,

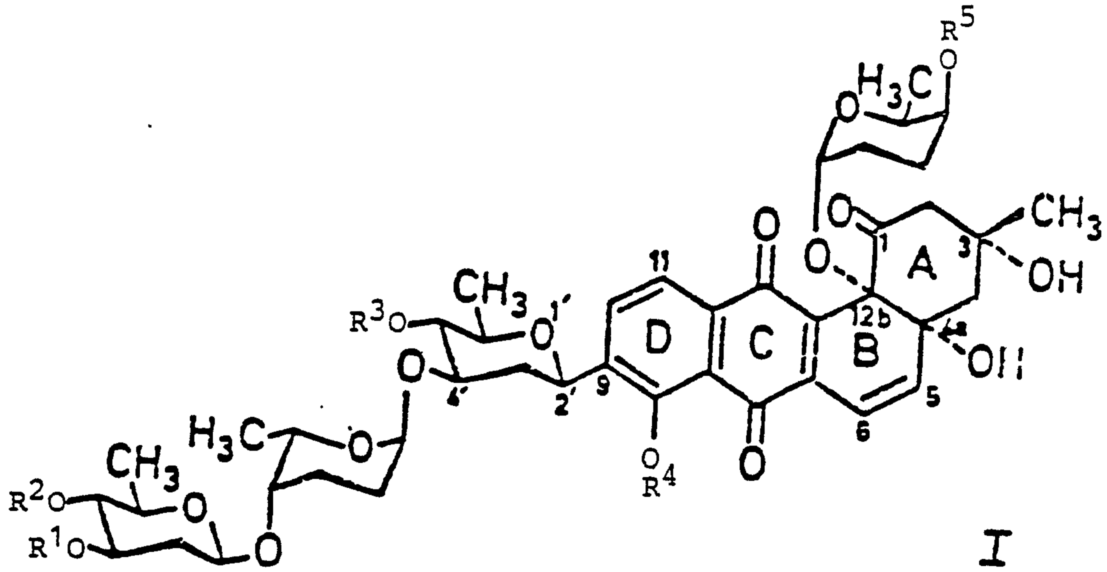

I

in der $R^1$ bis $R^5$ unabhängig voneinander Wasserstoff oder eine ($C_1$ bis $C_{18}$)-Acylgruppe ist, dadurch gekennzeichnet, daß 5',3B,4B,4C,8-Penta-O-acyl-urdamycin A basisch gespalten wird.

6. Verfahren nach Anspruch 5, dadurch gekennzeichnet, daß Penta-O-acyl-urdamycin A mit einer gesättigten Natriumcarbonatlösung in wäßrig/methanolischer Lösung gespalten wird.

7. Verwendung der Verbindung der allgemeinen Formel I erhältlich nach einem oder mehreren der Verfahren der Ansprüche 1 bis 6 zur Herstellung von Heilmitteln.

8. Verwendung der Verbindung nach Anspruch 7 zur Herstellung eines Heilmittels zur Behandlung von infektiösen Krankheiten und Tumoren.

9. Verwendung der Verbindung der allgemeinen Formel I erhältlich nach einem oder mehreren Verfahren der Ansprüche 1 bis 6 als antibiotisches Agens.